# EUROPEAN PATENT APPLICATION

(11) **EP 2 572 743 A1**
(43) Date of publication of application: **27.03.2013**
(21) Application number: 11182624.4
(22) Date of filing: 23.09.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a needle safety device (1) comprising a needle hub (1.1) having an inner surface (1.1.2) with a bearing element (1.1.1), a needle (1.2) coupled to the needle hub (1.1) and having a distal tip (1.2.1), a needle shield (1.3) telescopically coupled to the needle hub (1.1). The needle shield (1.3) comprises an outer sleeve (1.3.2) coupled to an inner sleeve (1.3.2) by a biasing element (1.3.1). In a first axial position (PA1), the needle shield (1.3) covers the distal tip (1.2.1) of the needle (1.2). In a second axial position (PA2), the needle shield (1.3) moves proximally relative to the needle hub (1.1) to expose the distal tip (1.2.1) of the needle (1.2), the outer sleeve (1.3.2) locks to the needle hub (1.1) and the biasing element (1.3.1) deforms around the bearing element (1.1.1). In a third axial position (PA3), the biasing element (1.3.1) expands, forcing the inner sleeve (1.3.3) distally relative to the needle hub (1.1) and covering the distal tip (1.2.1) of the needle (1.2).

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal safety needle assembly.

### Summary of the Invention

It is an object of the present invention to provide an improved safety needle assembly that minimizes the risk of an accidental needle stick injury, that is safe to handle, and that provides needle safety before and after the medicament is delivered.

In an exemplary embodiment, a needle safety device comprises a needle hub having an inner surface with a bearing element, a needle coupled to the needle hub and having a distal tip, and a needle shield telescopically coupled to the needle hub. The needle shield comprises an outer sleeve coupled to an inner sleeve by a biasing element. In a first axial position, the needle shield covers the distal tip of the needle. In a second axial position, the needle shield moves proximally relative to the needle hub to expose the distal tip of the needle, the outer sleeve locks to the needle hub and the biasing element deforms around the bearing element. In a third axial position, the biasing element expands, forcing the inner sleeve distally relative to the needle hub and covering the distal tip of the needle.

In an exemplary embodiment, the bearing element is an annular projection formed on a distal surface of the inner surface.

In an exemplary embodiment, the biasing element is a resilient deformable loop.

In an exemplary embodiment, distal end of the needle hub includes a flange and a locking protrusion proximal of the flange. The outer sleeve includes a first locking tab adapted to abut the flange in the first axial position and to abut the locking protrusion in the second axial position. The first locking tab and the locking protrusion have corresponding ramped surfaces. The first locking tab deflects radially when engaging the locking protrusion when the needle shield moves from the first axial position to the second axial position. The first locking tab and the locking protrusion prevent distal movement of the outer sleeve relative to the needle hub.

In an exemplary embodiment, the inner sleeve includes a second locking tab adapted to abut a distal face of the needle shield when in the third axial position. The first locking tab deflects radially when engaging the distal face when moving from the second axial position to the third axial position. The second locking tab and the distal face prevent proximal movement of the inner sleeve relative to the needle hub.

In an exemplary embodiment, the biasing element is integrally formed with the needle shield.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an isometric and sectional view of an exemplary embodiment of a needle safety device before use, wherein a needle shield is positioned in a first axial position;
- Figure 2: shows an isometric and sectional view of an exemplary embodiment of a needle safety device with a needle shield positioned in a second axial position;
- Figure 3: shows a sectional view of an exemplary embodiment of a needle safety device with a needle shield positioned in the second axial position;
- Figure 4: shows an isometric and sectional view of an exemplary embodiment of a needle safety device in a needle safe state;
- Figure 5: shows a sectional view of an exemplary embodiment of a needle safety device in a needle safe state;

Corresponding parts are marked with the same reference symbols in all figures.

### Detailed Description

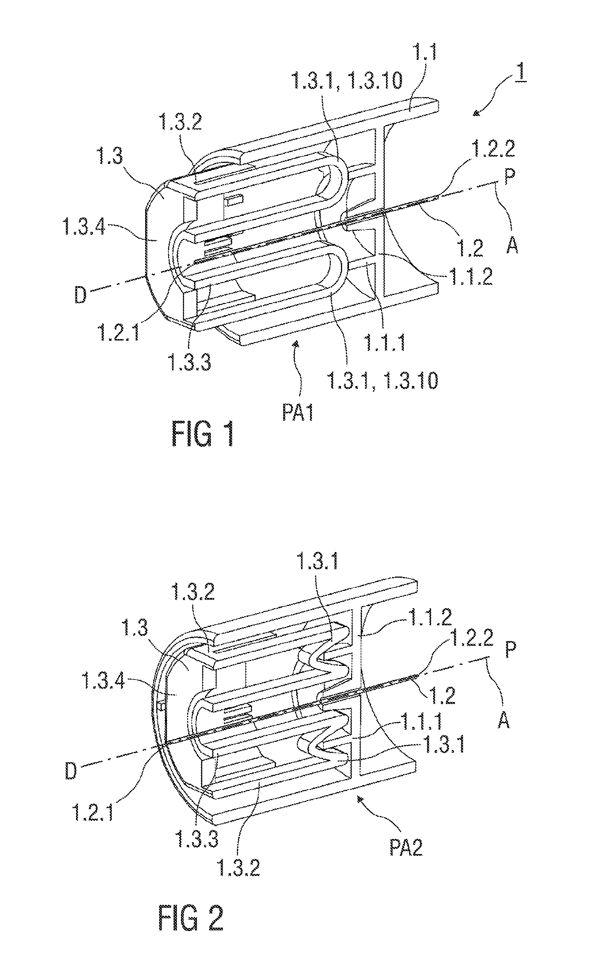

Figure 1 shows a sectional and isometric view of an exemplary embodiment of a needle safety device 1 according to the present invention. The safety device 1 comprises a needle hub 1.1, a needle 1.2 coupled to the needle hub 1.1, and a needle shield 1.3 coupled to the needle hub 1.1 and axially translatable relative to the needle hub 1.1.

In an exemplary embodiment, the needle hub 1.1 includes a proximal portion adapted to couple to an injection device, e.g., a syringe, a pen injector, an auto-injector, etc. For example, the proximal portion may include threads, a bayonet fit, a snap-fit arrangement, a friction-fit arrangement, etc. for coupling to the injection device. When the safety device 1 is coupled to the injection device, an inner surface 1.1.2 may abut a distal end of the injection device. The needle 1.2 may be coupled to the inner surface 1.1.2 and aligned with a longitudinal axis A of the safety device 1. A distal tip 1.2.1 of the needle 1.2 may extend through the needle shield 1.3, and a proximal tip 1.2.2 of the needle 1.2 may extend through the proximal portion of the needle hub 1.1 and into a container of medicament in the injection device (when the safety device 1 is coupled to the injection device). For example, the proximal tip 1.2.2 of the needle 1.2 may pierce a septum on the container to create a flow path for the medicament to be discharged from the injection device.

A bearing element 1.1.1 may be formed on a distal surface of the inner surface 1.1.2. In an exemplary embodiment, the bearing element 1.1.1 may be an annular wall formed on the distal surface of the inner surface 1.1.2.

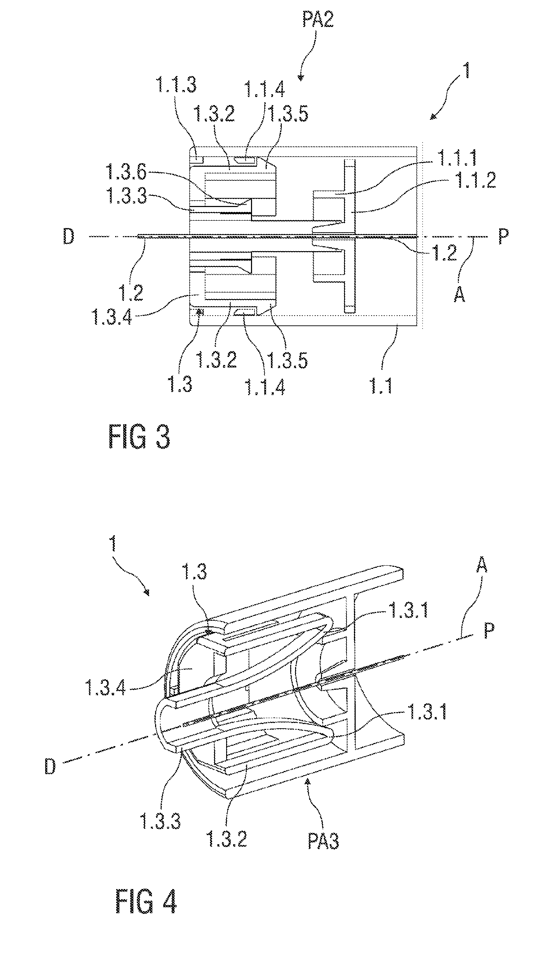

As shown in Figure 3, a distal end of the needle hub 1.1 may include an opening for receiving the needle shield 1.3. The distal end of the needle hub 1.1 may further include a flange 1.1.3. The flange 1.1.3 may be formed as an annular projection which projects toward the axis A of the safety device 1. Proximal of the flange 1.1.3 may be a locking protrusion 1.1.4 formed on the needle hub 1.1. The locking protrusion 1.1.4 may be formed as an annular projection which projects toward the axis A of the safety device 1.

Referring back to the exemplary embodiment shown in Figure 1, the needle shield 1.3 fits telescopically within the needle hub 1.1 and is axially translatable relative to the needle hub 1.1. In Figure 1, the needle shield 1.3 is shown in a first axial position (PA1) (pre-use) in which the distal tip 1.2.1 of the needle 1.2 is covered.

In an exemplary embodiment, the needle shield 1.3 may include one or more biasing elements 1.3.1 which are adapted to interact with the bearing element 1.1.1 on the inner surface 1.1.2 of the needle hub 1.1. In an exemplary embodiment, the biasing elements 1.3.1 are formed as deformable loops 1.3.10 formed integral with the needle shield 1.3 and adapted to abut the bearing element 1.1.1 when the needle shield 1.3 is in the first position. In an exemplary embodiment, the needle shield 1.3 may be formed as a single piece by, for example, injection molding. Extending distally from the biasing elements 1.3.1 are outer sleeve 1.3.2 and an inner sleeve 1.3.3. A distal portion of the outer sleeve 1.3.2 is coupled to a distal face 1.3.4, which lies in a plane normal to the axis A and is adapted to be pressed against an injection site.

As shown in the exemplary embodiment in Figure 3, an outer surface of the outer sleeve 1.3.2 includes a first locking tab 1.3.5 which interacts with the flange 1.1.3 and the locking protrusion 1.1.4. An outer surface of the inner sleeve 1.3.3 includes a second locking tab 1.3.6 which interacts with the distal face 1.3.4. These interactions are explained further below.

Referring back to the exemplary embodiment shown in Figure 1, the needle shield 1.3 is in the first axial position (PA1), and the safety device 1 is ready for an injection. The needle shield 1.3 is biased in the first axial position (PA1), because the biasing elements 1.3.1 abut the bearing element 1.1.1. The needle shield 1.3 is retained within the needle hub 1.1, because the first locking tab 1.3.5 abuts a proximal face of the flange 1.1.3.

Figure 2 shows an exemplary embodiment of the safety device 1 in a second axial position (PA2), e.g., during the injection. When the safety device 1 is pressed against an injection site, the needle shield 1.3 moves proximally into the needle hub 1.1, which exposes the distal tip 1.2.1 of the needle 1.2. As the needle shield 1.3 moves proximally, the biasing elements 1.3.1 compress against the bearing element 1.1.1. As shown in the exemplary embodiment in Figure 2, the deformable loops 1.3.10 abut and deform around the bearing element 1.1.1 to create stored energy given the resilience of the deformable loops 1.3.10.

Figure 3 shows an exemplary embodiment of the safety device 1 in the second axial position (PA2). As the needle shield 1.3 is moving from first axial position (PA1) to the second axial position (PA2), the first locking tab 1.3.5 on the outer sleeve 1.3.2 engages the locking protrusion 1.1.4 on the needle hub 1.1. When the first locking tab 1.3.5 engages the locking protrusion 1.1.4, the first locking tab 1.3.5 may deflect radially toward the axis A. Those of skill in the art will understand that corresponding ramped surfaces on the first locking tab 1.3.5 and the locking protrusion 1.1.4 may reduce the force necessary to deflect the first locking tab 1.3.5. When the needle shield 1.3 has reached the second axial position (PA2), the first locking tab 1.3.5 may return to its original (radial) position and engage a proximal abutment surface of the locking protrusion 1.1.4 which prevents distal displacement of the outer sleeve 1.3.2 relative to the needle hub 1.1. Those of skill in the art will understand that an axial space between the flange 1.1.3 and the locking protrusion 1.1.4 may be varied to vary the axial displacement of the needle shield 1.3 relative to the needle hub 1.1 which is necessary to lock the outer sleeve 1.3.2 relative to the needle hub 1.1.

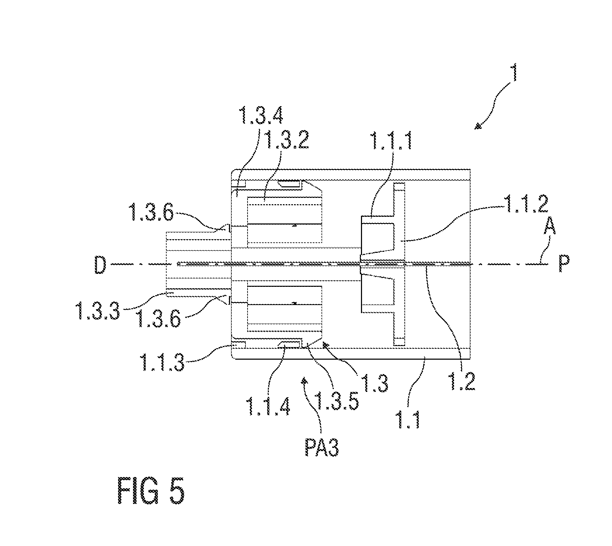

Figures 4 and 5 show an exemplary embodiment of the safety device 1 in a third axial position (PA3). When force is removed from the distal face 1.3.4 of the needle shield 1.3 (e.g., after an injection is complete), the biasing element 1.3.1 will deform around the bearing element 1.1.1. Because the outer sleeve 1.3.2 is locked relative to the needle hub 1.1, the biasing element 1.3.1 will force the inner sleeve 1.3.3 distally. As the inner sleeve 1.3.3 moves distally, the second locking tab 1.3.6 may engage a hole (for the needle 1.2) formed in the distal face 1.3.4. When the second locking tab 1.3.6 engages the hole, the second locking tab 1.3.6 may deflect radially toward the axis A until the second locking tab 1.3.6 has passed through the hole. Those of skill in the art will understand that corresponding ramped surfaces on the second locking tab 1.3.5 and the hole may reduce the force necessary to deflect the second locking tab 1.3.5. When the second locking tab 1.3.6 has passed through the hole, the second locking tab 1.3.6 may return to its original (radial) position and engage the distal face 1.3.4 which prevents proximal displacement of the inner sleeve 1.3.3 relative to the needle hub 1.1. Thus, in the third axial position (PA3) the safety device 1 is needle-safe, such that the distal tip 1.2.1 of the needle 1.2 will not be exposed if force is applied to the inner sleeve 1.3.3.

A peelable film (not shown) may be placed on the distal face 1.3.4 of the needle shield 1.3 to maintain sterility of the needle 1.2 prior to use.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle safety device (1) comprising:
a needle hub (1.1) having an inner surface (1.1.2) with a bearing element (1.1.1);
a needle (1.2) coupled to the needle hub (1.1), the needle (1.2) having a distal tip (1.2.1);
a needle shield (1.3) telescopically coupled to the needle hub (1.1), the needle shield (1.3) comprising an outer sleeve (1.3.2) coupled to an inner sleeve (1.3.2) by a biasing element (1.3.1),
wherein, in a first axial position (PA1), the needle shield (1.3) covers the distal tip (1.2.1) of the needle (1.2),
wherein, in a second axial position (PA2), the needle shield (1.3) moves proximally relative to the needle hub (1.1) to expose the distal tip (1.2.1) of the needle (1.2), the outer sleeve (1.3.2) locks to the needle hub (1.1) and the biasing element (1.3.1) deforms around the bearing element (1.1.1),
wherein, in a third axial position (PA3), the biasing element (1.3.1) expands, forcing the inner sleeve (1.3.3) distally relative to the needle hub (1.1) and covering the distal tip (1.2.1) of the needle (1.2).

2. The needle safety device (1) according to claim 1, wherein the bearing element (1.1.1) is an annular projection formed on a distal surface of the inner surface (1.1.2).

3. The needle safety device (1) according to any one of the preceding claims, wherein the biasing element (1.3.1) is a resilient deformable loop (1.3.10).

4. The needle safety device (1) according to any one of the preceding claims, wherein a distal end of the needle hub (1.1) includes a flange (1.1.3) and a locking protrusion (1.1.4) proximal of the flange (1.1.3).

5. The needle safety device (1) according to claim 4, wherein the outer sleeve (1.3.2) includes a first locking tab (1.3.5) adapted to abut the flange (1.1.3) in the first axial position (PA1) and to abut the locking protrusion (1.1.4) in the second axial position (PA2).

6. The needle safety device (1) according to claim 5, wherein the first locking tab (1.3.5) and the locking protrusion (1.1.4) have corresponding ramped surfaces.

7. The needle safety device (1) according to claim 5 or 6, wherein the first locking tab (1.3.5) deflects radially when engaging the locking protrusion (1.1.4) when the needle shield (1.3) moves from the first axial position (PA1) to the second axial position (PA2).

8. The needle safety device (1) according to claims 5, 6 or 7, wherein the first locking tab (1.3.5) and the locking protrusion (1.1.4) prevent distal movement of the outer sleeve (1.3.2) relative to the needle hub (1.1).

9. The needle safety device (1) according to any one of claims 1-4, wherein the inner sleeve (1.3.3) includes a second locking tab (1.3.6) adapted to abut a distal face (1.3.4) of the needle shield (1.3) when in the third axial position (PA3).

10. The needle safety device (1) according to claim 8, wherein the first locking tab (1.3.5) deflects radially when engaging the distal face (1.3.4) when moving from the second axial position (PA2) to the third axial position (PA3).

11. The needle safety device (1) according to claims 9 or 10, 5 or 6, wherein the second locking tab (1.3.6) and the distal face (1.3.4) prevent proximal movement of the inner sleeve (1.3.3) relative to the needle hub (1.1).

12. The needle safety device (1) according to any of the preceding claims, wherein the biasing element (1.3.1) is integrally formed with the needle shield (1.3).
